(19) Europäisches Patentamt

European Patent Office

Office européen des brevets

(11) **EP 0 701 803 B1**

(12) **EUROPEAN PATENT SPECIFICATION**

(45) Date of publication and mention
of the grant of the patent:
**06.10.1999 Bulletin 1999/40**

(51) Int. Cl.$^6$: **A61F 2/30**, A61B 17/58

(21) Application number: **95100829.1**

(22) Date of filing: **23.01.1995**

(54) **Medical device for implantation into living bodies**

Medizinische Vorrichtung zur Implantierung in ein Lebewesen

Dispositif médical implantable dans des organismes vivants

(84) Designated Contracting States:
**CH DE ES FR GB IT LI**

(30) Priority: **03.02.1994 US 306968**

(43) Date of publication of application:
**20.03.1996 Bulletin 1996/12**

(73) Proprietor: **SYNTHES AG Chur
7002 Chur (CH)**

(72) Inventors:
• **Tepic, Slobodan
CH-7270 Davos (CH)**

• **Richards, Robert Geoffrey
Dyfed, Wales (GB)**

(74) Representative:
**Lusuardi, Werther Giovanni, Dr.
Dr. Lusuardi AG,
Kreuzbühlstrasse 8
8008 Zürich (CH)**

(56) References cited:
EP-A- 0 359 575      EP-A- 0 388 576
EP-A- 0 610 837      WO-A-93/00870
DE-A- 3 116 040      US-A- 4 767 418
US-A- 4 865 603

## Description

This invention relates to a medical device for implantation into living bodies according to the preamble of claim 1.

Postoperative infections still play an important role in jeopardising the result of surgical treatment of bone fractures and joint disease. The relative incidence of infections is 1 - 2 % at least.

It is evident that resistance to infection (provided good implant materials are used) at the implant/soft tissue interface is determined by the strength of adhesion of the connective tissue to the implant. The extremities of the situation are:

1. Interface motion causing the formation of a fibrous capsule, surrounding the implant with a continuous liquid phase favouring the spread of bacterial infection and impeding the mobile cellular defense of the body.

2. Strong adhesion of the soft tissue to the implant allows minimal fibrous tissue formation, thus confining a bacterial contamination. Close contact of blood vessels to the implant improves the body's defense. The draw back of strong adhesion of the soft tissue to the implant is that it can cause tissue trauma during removal of the implant after fracture healing.

The invention as claimed is intended to cure these problems of too much or too little adhesion of the soft tissues to the implant. Varying the degree of rough/smooth surface area will allow the problems to be solved in different parts of the body.

The invention will have numerous applications in the body for all implant plates and most prosthesis. Variations of the invention include the following:

1. A network of fine rough surface with smooth compartments in between covering at least partially the surface of the prosthesis or implant.

2. A raised network of fine rough surface with smooth compartments below in between the network covering at least partially the surface of the prosthesis or implant.

The rough surface may be made by etching with an acid or mixture of acids as for example with standard cleaning of an implant plate. If a raised rough surface was to be used in combination with a lower smooth surface the pattern could be stamped onto the implant and the lower surface protected before making the raised pattern rough with etching. The rough / smooth surface could also be made by photoetching of a thin foil to produce the network followed by acid etching of the network produced. The foil would then be bonded onto the smooth surface of the implant, giving a raised rough surface.

The design contains numerous additional advantages to those stated in the initial problem for minimising possibility of bacterial infection.

In the example of the raised network, the fine rough surface protects the initial invading cells, such as fibroblastics cells, from shear forces from body fluid motion during the important initial stages of cell attachment and proliferation. The smooth surface promotes cell spreading and early attachment of the cells therefore accelerating the cell cycle with the consequence of increasing proliferation of cells. The raised network also separates compartments of smooth surface which prevents bacteria from spreading from one compartment to the next. When the soft tissue comes to rest on the implant the rough surface will enable optimal adhesion of the tissue to the implant via the tissue's extracellular matrix and in the case of raised rough surface the compartments which will have been filled by invading cells will act as plugs of tissue which will attach to the overlying soft tissue and prevent motion of the soft tissue over the implant or prosthesis, preventing tissue trauma.

Examples of the use of the rough and smooth surface would include plates with this combination surface on the uppersurface of the plate where it is in contact with the soft tissue. The rough and smooth surface would be beneficial to all metallic plates and also to biodegradable ones. In the case of any biodegradable plates and other implants or prosthesis' the amount of rough surface compared to the amount of smooth surface could be increased since the implant or prosthesis should degrade into the body and would not need to be taken out. Therefore the amount of adhesion could be allowed to be much greater than for an implant or prosthesis that would have to be taken out. A pacemaker prosthesis could have the combination rough and smooth surface on all of it's area in contact with living tissue to promote good adhesion and minimise the chance of infection.

The various features of novelty which characterize the invention are pointed out with particularity in the claims annexed to and forming part of this disclosure. For the better understanding of the invention, its operating advantages and specific objects attained by its use, reference should be had to the accompanying drawings, examples and descriptive matter in which are illustrated and described preferred embodiments of the invention.

In the drawings:

Fig. 1 is a cross section of a conventional implant or prosthesis having either a rough or smooth surface, but not a combination of both;

Fig. 2 is a cross section of an implant of prosthesis with the invention of a combination of rough and smooth areas both on the same surface of the implant or prosthesis;

Fig. 3 is a cross section of an implant or prosthesis with the invention of a combination of a raised rough surface separating compartments of smooth surface in between; and

Fig. 4 is an example of the rough and smooth surface combined as applied to an implant plate from an uppersurface view.

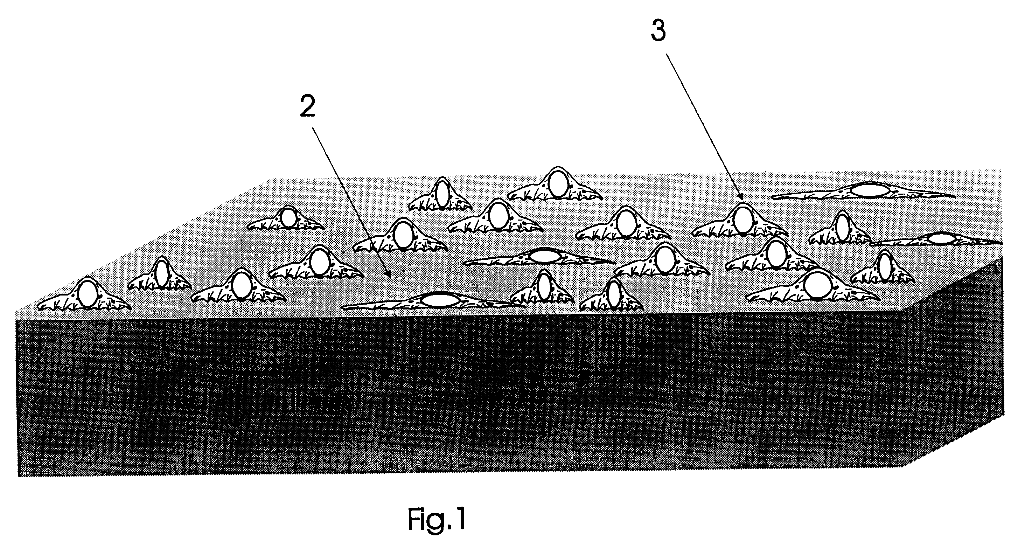

Figure 1 shows a prior art bone plate 1 having an uppersurface 2 of uniform roughness. Roughness can be either very low as with polished plates or can be significant as in the case of sandblasted or etched bone plates. The cell, e.g. fibroblasts 3 will adhere to the uppersurface 2 of the bone plate 1 according to its roughness.

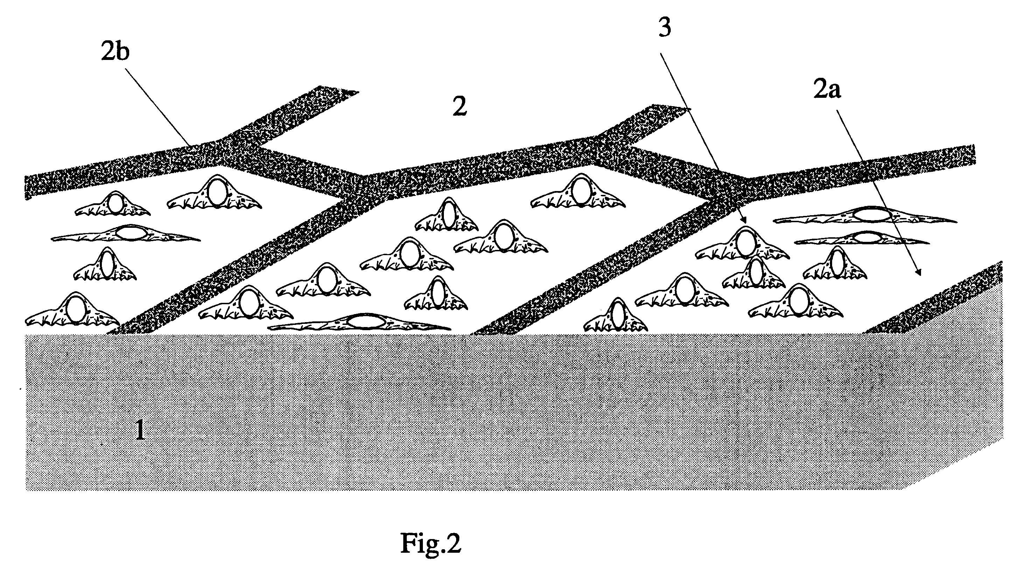

In Fig. 2 the uppersurface 2 of the bone plate 1 according to the invention is divided into first and second types of surface areas with different roughness. The first type of surface area consists of a plurality of isle-like surface portions 2a of lower roughness surrounded by the second type of surface area consisting of a continuously interconnected surface portion 2b of higher roughness, in the form of a network, for example of hexagonal structure. The total surface area of lower roughness is equal to $A_1 = \Sigma a_j$, where $a_j$ is the surface area of the individual isle-like surface portions. The total surface of the network of higher roughness is equal to $A_2$. The control of the tissue adhesion is possible by changing the ratio of $A_1/A_2$ which typically is in the region of 1 - 10, preferably 2 - 5.

The smooth surface portions 2a promote spreading and early attachment of the fibroblasts 3.

The function of the rougher surfaces 2b of the network is to provide for stronger attachment of the extracellular matrix (collagen).

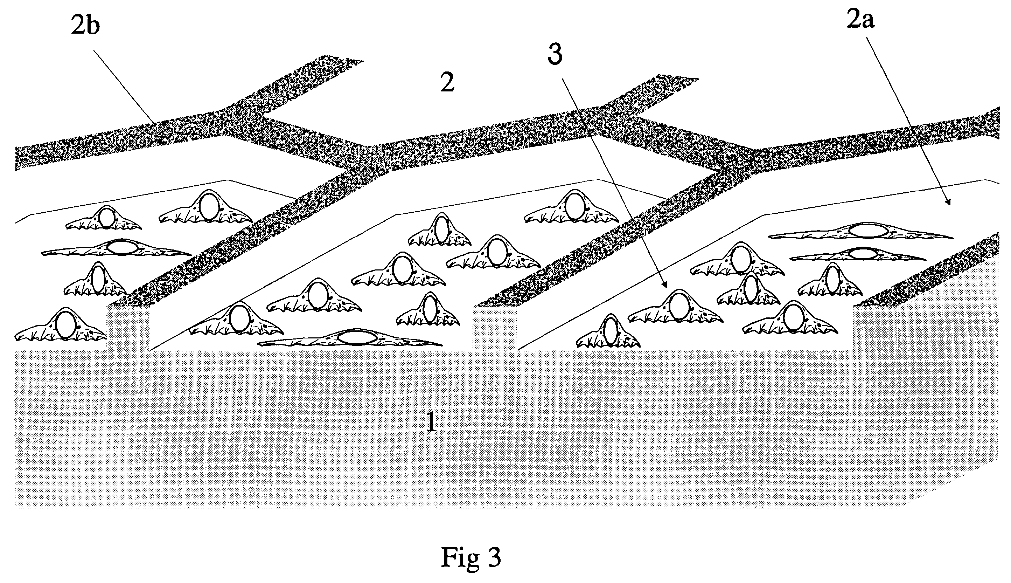

Fig. 3 shows a preferred embodiment of the invention in which the uppersurface 2 of the bone plate 1 is divided in the same manner as in the example of Fig. 2 but where the rough surface portion 2b is raised with respect to the surface portions 2a of lower roughness, thereby separating the latter to form compartments of smooth surface in between the rough surface portion 2b. The surface portion 2b of higher roughness protects the fibroblastic cells 3 initially invading the lower smooth compartments of surface portions 2a.

The preferred method of producing such a surface is firstly acid etching of the complete surface, e.g. with hydrofluoric acid in the case of titanium which will lead to about 1 μ RMS (i.e. root mean square) roughness and secondly stamping the etched surface with a patterned tool. The stamping of the lowered regions will lead to a significant decrease in roughness, e.g. stamping to a depth of about 100 μ reduced the RMS value of the roughness to about 0.4 μ.

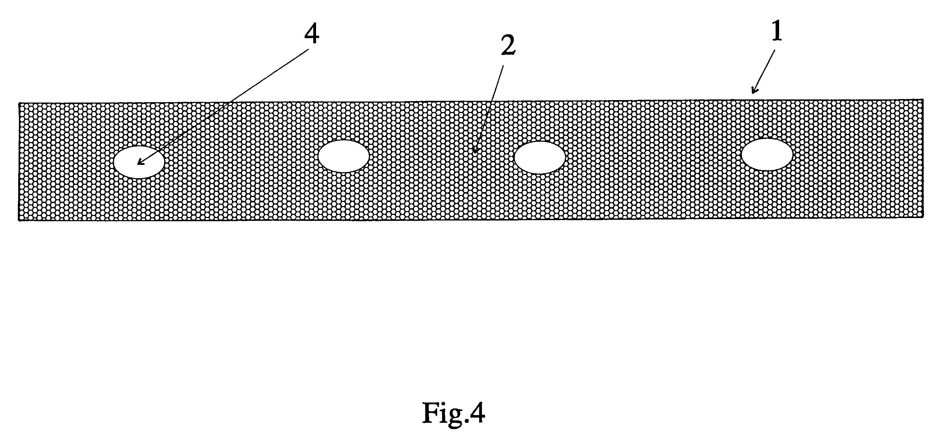

Fig. 4 shows the total upperside 2 of a bone plate 1 with holes 4 having the inventive combination of portions of lower roughness 2a surrounded by a surface portion of higher roughness 2b as shown in detail in Fig. 3.

While the foregoing description and drawings represent the preferred embodiments of the present invention, it will be obvious for those skilled in the art that various changes and modifications may be made therein without departing from the scope of the present invention.

## Claims

1. Medical device for implantation into living bodies having first and second types of surface areas with different roughness, wherein the first type of surface area consists of a plurality of isle-like surface portions (2a) of lower roughness surrounded by the second type of surface area consisting of a continuously interconnected surface portion (2b) of higher roughness characterised in that the total surface area of the first type $A_1 = \Sigma a_j$, $a_j$ being the surface area of the isle-like surface portions, is at least equal or larger than the total surface area of the second type $A_2$.

2. Medical device according to claim 1, wherein the ratio $A_1/A_2$ between the total surface area of the first type $A_1 = \Sigma a_j$, $a_j$ being the surface area of the isle-like surface portions, to the total surface area of the second type $A_2$ is in the range between 1 - 10, preferably 2 - 5.

3. Medical device according to claim 1 or 2, wherein the RMS (root mean square) roughness of the said first type of surface area is less than 1.0 μ, preferably less than 0.5 μ.

4. Medical device according to claim 1 or 2, wherein the RMS (root mean square) roughness of the said second type of surface area is larger than 0.5 μ, preferably larger than 1.0 μ.

5. Medical device according to one of the claims 1 - 4, wherein the roughness of the said second type of surface area is at least two times higher than the roughness of the said first type of surface area.

6. Medical device according to one of the claims 1 - 5, wherein said plurality of discontinuous surface portions of the first type of surface area are in the form of regular polygons, preferably squares or hexagons.

7. Medical device according to one of the claims 1 - 6, wherein first type of surface area is located at a lower level than second type of surface area, said plurality of discontinuous surface portions of lower roughness forming depressions in said continuous surface portion of higher roughness.

8. Medical device according to one of the claims 1 - 7, wherein the distance between the levels of said first and second types of surface areas is in the range of 5 - 200 μm, preferably 30 - 80 μm.

9. Medical device according to one of the claims 1 - 8, wherein the surface $a_j$ of one of said discontinuous surface portions of said first type of surface area is larger than 0.1 mm$^2$.

10. Medical device according to one of the claims 1 - 8, wherein the surface $a_j$ of one of said discontinuous surface portions of said first type of surface area is smaller than 10 mm$^2$.

**Patentansprüche**

1. Medizinische Vorrichtung zur Implantierung in einen lebenden Körper, welche erste und zweite Arten von Oberflächengebieten mit unterschiedlicher Rauhigkeit aufweist, wobei die erste Art von Oberflächengebieten aus einer Vielzahl von inselartigen Oberflächenteilen (2a) mit geringerer Rauhigkeit besteht, umgeben durch die zweite Art von Oberflächengebiet, welche aus einem kontinuierlich verbundenen oberflächenteil (2b) mit einer höheren Rauhigkeit besteht, dadurch gekennzeichnet, dass das gesamte Oberflächengebiet der ersten Art $A_1 = \Sigma a_j$, worin $a_j$ die Oberflächengebiete der inselartigen Oberflächenteile sind, mindestens gleich gross oder grösser als das gesamte Oberflächengebiet der zweiten Art $A_2$ ist.

2. Medizinische Vorrichtung nach Anspruch 1, dadurch gekennzeichnet, dass das Verhältnis $A_1/A_2$ zwischen den gesamten Oberflächengebieten der ersten Art $A_1 = \Sigma a_j$, worin die $a_j$ die Oberflächengebiete der inselartigen Oberflächenteile sind, zum gesamten Oberflächengebiet der zweiten Art $A_2$ im Bereich zwischen 1 - 10, vorzugweise 2 - 5 liegt.

3. Medizinische Vorrichtung nach Anspruch 1 oder 2, dadurch gekennzeichnet, dass der RMS-Wert (root mean square) der Rauhigkeit der erwähnten ersten Art von Oberflächengebieten weniger als 1,0 μ, vorzugsweise weniger als 0,5 μ beträgt.

4. Medizinische Vorrichtung nach Anspruch 1 oder 2, dadurch gekennzeichnet, dass der RMS-Wert (root mean square) der Rauhigkeit der erwähnten zweiten Art von Oberflächengebiet grösser als 0,5 μ, vorzugsweise grösser als 1,0 μ ist.

5. Medizinische Vorrichtung nach einem der Ansprüche 1 bis 4, dadurch gekennzeichnet, dass die Rauhigkeit der erwähnten zweiten Art von Oberflächengebiet mindestens doppelt so gross ist als die Rauhigkeit der erwähnten ersten Art von oberflächengebieten.

6. Medizinische Vorrichtung nach einem der Ansprüche 1 bis 5, dadurch gekennzeichnet, dass die erwähnte Vielzahl von diskontinuierlichen Oberflächengebieten der ersten Art die Form von regulären Polygonen, vorzugsweise Vierecken oder Sechsecken haben.

7. Medizinische Vorrichtung nach einem der Ansprüche 1 bis 6, dadurch gekennzeichnet, dass die erste Art von Oberflächengebieten auf einem tieferen Niveau angeordnet ist als die zweite Art von Oberflächengebiet, so dass die erwähnte Vielzahl von diskontinuierlichen Oberflächenteilen mit geringerer Rauhigkeit Vertiefungen im erwähnten kontinuierlichen Oberflächenteil mit grösserer Rauhigkeit bilden.

8. Medizinische Vorrichtung nach einem der Ansprüche 1 bis 7, dadurch gekennzeichnet, dass die Distanz zwischen den Niveaus der erwähnten ersten und zweiten Art von Oberflächengebieten im Bereich von 5 - 200 μm, vorzugsweise 30 - 80 μm ist.

9. Medizinische Vorrichtung nach einem der Ansprüche 1 bis 8, dadurch gekennzeichnet, dass die Oberfläche $a_j$ eines der erwähnten diskontinuierlichen Oberflächenteils der erwähnten ersten Art von Oberflächengebiet grösser als 0,1 mm$^2$ ist.

10. Medizinische Vorrichtung nach einem der Ansprüche 1 bis 8, dadurch gekennzeichnet, dass die Oberfläche $a_j$ eines der erwähnten diskontinuierlichen Oberflächenteils der erwähnten ersten Art von Oberflächengebiet kleiner als 10 mm$^2$ ist.

**Revendications**

1. Dispositif médical destiné à être implanté dans des organismes vivants et présentant un premier et un deuxième type de surface de rugosités différentes, dans lequel le premier type de surface est constitué de plusieurs parties de surface (2a) en forme d'îlots, de plus faible rugosité, entourées par le deuxième type de surface constituées de parties de surface (2b) de plus forte rugosité reliées de manière continue, caractérisé en ce que la superficie totale du premier type $A_1 = \Sigma a_j$, $a_j$ étant la superficie des parties de surface en forme d'îlots, est au moins égale ou supérieure à la superficie totale du deuxième type $A_2$.

2. Dispositif médical selon la revendication 1, dans lequel le rapport $A_1/A_2$ entre la superficie totale du premier type $A_1 = \Sigma a_j$, $a_j$ représentant la superfi-

cie des parties de surface en forme d'îlots et la superficie totale du deuxième type $A_2$ est compris dans la plage de 1 à 10, et de préférence de 2 à 5.

3. Dispositif médical selon les revendications 1 ou 2, dans lequel la rugosité en moyenne quadratique dudit premier type de surface est inférieure à 1,0 $\mu$, et de préférence inférieure à 0,5 $\mu$.

4. Dispositif médical selon les revendications 1 ou 2, dans lequel la rugosité en moyenne quadratique dudit deuxième type de surface est supérieure à 0,5 $\mu$, et de préférence supérieure à 1,0 $\mu$.

5. Dispositif médical selon l'une des revendications 1 à 4, dans lequel la rugosité dudit deuxième type de surface est au moins deux fois plus élevée que la rugosité dudit premier type de surface.

6. Dispositif médical selon l'une des revendications 1 à 5, dans lequel ladite pluralité de parties de surface discontinues du premier type de surface présente la forme de polygones réguliers, de préférence des carrés ou des hexagones.

7. Dispositif médical selon l'une des revendications 1 à 6, dans lequel le premier type de surface est situé à un niveau plus bas que le deuxième type de surface, ladite pluralité de parties de surfaces discontinues de plus faible rugosité formant des creux dans ladite partie continue de surfaces de rugosité plus élevée.

8. Dispositif médical selon l'une des revendications 1 à 7, dans lequel la distance entre les niveaux dudit premier et dudit deuxième types de surface est comprise dans la plage de 5 à 200 $\mu$m, de préférence de 3 à 80 $\mu$m.

9. Dispositif médical selon l'une des revendications 1 à 8, dans lequel la surface $a_j$ de l'une desdites parties discontinues de surfaces dudit premier type de surface est supérieure à 0,1 mm$^2$.

10. Dispositif médical selon l'une des revendications 1 à 8, dans lequel la surface $a_j$ de l'une desdites parties de surfaces discontinues dudit premier type de surface est inférieure à 10 mm$^2$.

Fig.1

Fig.2

EP 0 701 803 B1

Fig 3

Fig.4